# EUROPEAN PATENT SPECIFICATION

(11) **EP 0 888 116 B1**
(45) Date of publication and mention of the grant of the patent: **10.01.2001**
(21) Application number: 97920642.2
(22) Date of filing: 10.04.1997
(51) Int. Cl.: A61K 31/565

(54) **USE OF DEHYDROEPIANDROSTERONE FOR THE TREATMENT OF ADDICTIONS**
VERWENDUNG VON DEHYDROEPIANDROSTERON ZUR SUCHTBEHANDLUNG
UTILISATION DE DEHYDRO-EPIANDROSTERONE DANS LE TRAITEMENT DES ETATS DE DEPENDANCE

(30) Priority: 10.04.1996 NL 1002833
(43) Date of publication of application: 07.01.1999
(73) Proprietor: Nefkens, Gerardus Joannes Emile Maria, 3067 JH Rotterdam (NL)
(72) Inventor: NEFKENS, Gerardus, Joannes, Emile, Maria, NL-3067 JH Rotterdam (NL); NOIJEN, Helga, Manuela, NL-3067 JH Rotterdam (NL)
(86) International application number: PCT/EP97/01779
(87) International publication number: WO 97/37663

(56) References cited:
- US-A- 4 518 595
- S. SCHWARZ ET AL.: "Steroids and opiate receptors." J. STEROID BIOCHEM., vol. 48, no. 4, 1994, pages 391-402, XP002020985 cited in the application
- B. BUDZISZEWSKA ET AL.: "Neurosteroids and the naloxone-precipitated withdrawal syndrome in morphine dpendent mice." EUR. NEUROPSYCHOPHARMACOL., vol. 6, no. 2, 1996, pages 135-140, XP000612742
- F. FACCHINETTI ET AL: "Reduced estriol and dehydroepiandrosterone sulphate plasma levels in methadone-addicted pregnant women." EUR. J. OBSTET. GYNECOL., vol. 23, no. 1-2, 1986, pages 67-73, XP000612727

## Description

The present invention is concerned with a method for the treatment of addicted human beings and the use of specific steroids for that purpose.
Particularly the invention relates to the treatment of nicotine-addicted people and the use of sex-steroid precursors for that purpose.

Smoking of tobacco may cause cancer and vascular diseases. Particularly consumption of nicotine may cause arteriosclerosis, heartattacks, cerebral strokes, etc. etc.
So far, no single effective method has been developed for the treatment of nicotine addicted humans and no effective pharmaceuticals have been disclosed, usable for the treatment of humans, addicted to nicotine or other addicting products.
Recent nicotine containing anti-smoke treatments do increase nicotine levels and the above dangers for users who, regardless the prescription, continue smoking during treatment, adding nicotine on nicotine.

According to the present invention addicted humans can be cured by administering, bringing into the bloodstream in any way whatsoever, of an effective amount of sex steroid precursors or an analogue thereof.
The invention also relates to the use of sex steroid precursors, for preparing a medicine, intended for the therapeutic use c.q. the treatment of addicted humans beings, for instance dependance of nicotine caused by smoking of tobacco, gluing of nicotine stamps on the skin, plasters, using nicotine inhalers, sprays, injections, etc.
Similary, the invention can be used for the treatment of other forms of addiction caused by the consumption of material or foods, alien to the human body like coffee, tea, alcohol, etc. and all kind of drugs.

The sex steroid precursors dehydroepiandrosterone (DHEA) and dehydroepiandrosteronsulfate (DHEAS) are major natural adrenal secretory products in humans. They are well known substances, which in the body are converted into active hormones like testosteron and estrogen, which substances are essential for many critical functions such as regulating libido, metabolism, etc. Since these compounds are converted in the body into active hormones they are indicated in this specification as precursors. DHEA and DHEAS are natural products but can also be prepared synthetically.
Analogues of the above mentioned precursors are synthetically prepared molecules eg. fluoro- or bromo-epiandrosteron. Certain analogues can be converted in vivo into one of the above mentioned precursors, their active componants or hormones.

If within the scope of this specification reference is made to DHEA, the other above mentioned precursors and analogues are included as well.

The plasma concentration of DHEAS which is the second abundant steroid in humans, undergoes the most marked age-related decline of any known steroid.
In the body of a 25 year old human DHEAS is present in abundance. In micrograms per milliliter up to about 4.5 for female and 7.9 for man. After this age, the concentration lowers down to about 10% at the age of 80.

The body uses these precursors to create sex hormones, but in many publications it is presumed that these precursors are also engaged in other body-activities.

Numerous publications are dedicated to the possible effects of the application of DHEA, also in some patent publications various therapeutic effects of its administration are pointed out, for instance :
as anti-carcinogen : EP-0627921.
for utilization with cancer of the uterus : WO-9416709.
with lupus erythematosus : WO-9408589 and
eye diseases : WO-9404155.

Furthermore literature exists concerning treatment of breast cancer (Lancet2-395-398-1971),
and concerning obesity.
Concerning blood concentrations of twenty different steroids in relation to the concentration of the interneural fluid low affinity interaction of steroids with neuronal membrane-bound receptors (J.Steroid Biochem, Molec.Biol. Vol48.No4. 1994),
about the lower ESTRIOL and DHEAS level noticed in pregnant methadone addicted woman (Eur. J. Obstet Gynecol.,23. pages 67-73) 1986) . It was concluded that methadone (and probably heroine) use during pregnancy was accompanied by a reduction of circulating DHEAS levels. Although a link seems to exist between DHEA and methadone, it has never been suggested that administering DHEA might cure methadone or heroin addiction, neither nicotine or other food related addiction.
Surprisingly it has been observed that addiction to nicotine, or other addictive drugs can be cured by administering or in whatever way bringing in the blood of an addicted person an effective amount of DHEA: equally, has been established that DHEA can be used for the preparation of a medicine, intended for therapeutic use, aimed at the detoxication from addicting substances particulary nicotine.
It was found that an overdose of DHEA will have a maximum effect, which will not necessarily mean that the level of DHEA in the blood should be brought at, or higher than, youth related levels.

It is pointed out that for each individual the proper level of DHEA must be established, which will be for most of the time a level that is higher than generally present in the blood for the persons at that age.

The advantage of the invention is that if the right person-depending quantity will be administrated, the detoxication process will be easy and painless.

The effectiveness of the present invention can easily be demonstrated at short notice by treatment of addicted persons, choosen at random.

Another aspect of the invention concerns food or refreshments fortified with an effective amount of DHEA , preferably foodstuffs that are industrially prepared and normally consumed on a daily basis eg. chewing gum, candy, soup, margarine or reduced-fat varieties .

In whatever way use shall be made of the present invention, no risk exist that the former addicted person will unvoluntarily return to his bad habit for if he chooses to do so, he can just start another treatment according to this invention.

From now on. those who want or not want to continue a certain consumption, can do so by a decision based on free will.

It has been observed that this invention works out differently per addicting product and differently per person.
While in respect of nicotine a certain disgust against smoking will be developped. the effects on cafeine and alcohol are milder and softer, creating a certain indifference.
Furthermore, as the invention will have a beneficial effect on general health. apart from diminishing lots of suffering, it will generate major savings on state health-care budgets.

The positive effects of the invention will come quicker by (partial) oral sublingual consumption of the appropriate amount of the age-dependent mentioned 'overdose'. varying from 100 up to 300 or even more milligram per day, like 10 times per day 30 milligram.

Regular medical control is advisable including bloodanalysis. to ensure the absence of high levels of prostate specific antigen (PSA check) and it could be advantageous to combine the treatment with antioxidants like the vitamins E. C, and Coenzymes.

The precursors themselves mentioned above can have a slight addicting effect. However gradual reduction of the intake to avoid another addiction will not cause problems comparable to nicotine addiction. Furthermore, sex steroide precursors are in all liturature considered harmless for human beings with acceptable PSA levels.

The positive effects could be diminished by parallel intake of hormones and possibly by the consumption of meat, because of a possible presence of non- or anti-coherent hormones.

This is another reason to have the invention applied under medical control, which at the same time will ensure a safeguard in respect of the quality and origin of the precursor(s) to he administered.

### EXAMPLE:

Man, age 59, 1.80M, 54 kilo, healthy, hectic lifestyle, was smoking during 40 years some 20 up to at last 70 cigarettes a day and his coffee consumption was on an average of 1 liter a day.

He did numerous unsuccessfull trials to stop both habits.

He started on april the 19th, 1995 after a medical check-up including blood (PSA) analysis with oral sublingual intake of 300 nig DHEA per day
(10 times 30mg).
After the first day of treatment already a slight but tracable indifference for cigarettes and consumption of coffee was observed.
After weeks Tea, chocolat, sugar and alcohol lost some of their attraction.
After 3 weeks a strong reduction of cigarette consumption resulted without any effort and after some 8 weeks followed resentment.
During the first year, abrupt stopping of the DHEA intake (performed as a test about 5 times) brought back the grieving for the mentioned products, but less and less so. Concerning coffee and other products the result was indifference.

After 10 months the intake of DHEA was reduced to 200 milligram per day.
After 15 months to 100 milligram per day in about 6 or 7 sublingual pills of 15 milligram.
In general the sublingual DHEA intake occurred on moments when formerly a cigarette would have been taken. During the months less and less of DHEA was sufficient.
After almost 2 years of almost finished treatment (actually 5 milligram DHEA per day is taken), the important difference with persons who quitted smoking just by will power or following another method. Neither cigarettes nor their smell have any inviting value.
On the contrary, coffee is diminished to 2 cups a day without problem.

## Claims

1. Use of DHEA, DHEAS, its precursors or analogues for preparing a medicament for curing addicted human beings.

2. Use according to claim 1 wherein the medicament is for treating human beings addicted to nicotine.

3. Use according to claim 1 wherein the medicament is for treating human beings addicted to alcohol.

4. Use according to claim 1 wherein said human beings have a drug consumption-related addiction.

5. Use according to claim 4 wherein the addictive drug is heroine or methadone.

6. Use according to claim 1 wherein said human beings have a food consumption-related addiction.

7. Use according to claim 6 wherein the food is coffee, tea, sugar or chocolate.

8. Use according to claim 1 wherein the medicament is mixed with the food diet of the addicted human being.

## Patentansprüche

1. Verwendung von DHEA, DHEAS, oder deren Vorläufern oder Analogen zur Herstellung eines Medikamentes zur Behandlung von süchtigen Menschen.

2. Verwendung nach Anspruch 1, wobei das Medikament zur Behandlung von nicotin-süchtigen Menschen ist.

3. Verwendung nach Anspruch 1, wobei das Medikament zur Behandlung von alkohol-süchtigen Menschen ist.

4. Verwendung nach Anspruch 1, wobei das Medikament zur Behandlung von drogen-süchtigen Menschen ist.

5. Verwendung nach Anspruch 4, wobei die Droge Heroin oder Methadon ist.

6. Verwendung nach Anspruch 1, wobei das Medikament zur Behandlung von nahrungsmittel-süchtigen Menschen ist.

7. Verwendung nach Anspruch 6, wobei das Nahrungsmittel Kaffee, Tee, Zucker oder Schokolade ist.

8. Verwendung nach Anspruch 1, wobei das Medikament mit der Nahrung des süchtigen Menschen gemischt wird.

## Revendications

1. Utilisation de la DHEA, de la DHEAS ou de ses précurseurs ou analogues pour la préparation d'un médicament pour traiter la dépendance chez les êtres humains.

2. Utilisation selon la revendication 1 où le médicament est pour le traitement de la dépendance à la nicotine.

3. Utilisation selon la revendication 1 où le médicament est pour le traitement de la dépendance à l'alcool.

4. Utilisation selon la revendication 1 oû les être humains ont une dépendance à liée à la consommation de drogue.

5. Utilisation selon la revendication 4 où la drogue est l'héroïne ou la méthadone.

6. Utilisation selon la revendication 1 oû les être humains ont une dépendance à liée à la consommation de nourriture.

7. Utilisation selon la revendication 6 où la nourriture est le café, le thé, le sucre ou le chocolat.

8. Utilisation selon la revendication 1 où le médicament est mélangé à la nourriture de l'être humain dépendant.
